# EUROPEAN PATENT SPECIFICATION

(11) **EP 2 240 154 B1**
(45) Date of publication and mention of the grant of the patent: **23.04.2014**
(21) Application number: 09706419.0
(22) Date of filing: 21.01.2009
(51) Int. Cl.: A61K 8/73, A61Q 19/00

(54) **COSMETIC COMPOSITION COMPRISING GLYCOGEN FOR SKIN APPLICATION WITH VELVET EFFECT**
KOSMETISCHE ZUSAMMENSETZUNG MIT GLYKOGEN ZUR HAUTAPPLIKATION MIT SAMTIGER WIRKUNG
COMPOSITION COSMÉTIQUE COMPRENANT UN GLYCOGÈNE POUR UNE APPLICATION SUR LA PEAU AVEC UN EFFET DE VELOURS

(30) Priority: 01.02.2008 EP 08425060
(43) Date of publication of application: 20.10.2010
(73) Proprietor: Aziende Chimiche Riunite Angelini Francesco A.C.R.A.F. S.p.A., 00181 Roma (IT)
(72) Inventor: MARCHITTO, Leonardo, I-62017 Porto Recanati (MC) (IT); RAGNI, Lorella, I-60033 Chiaravalle (AN) (IT); MARIOTTI, Francesca, I-61100 Pesaro (PU) (IT)
(74) Representative: Allaix, Roberto
(86) International application number: PCT/EP2009/050632
(87) International publication number: WO 2009/095341

(56) References cited:
- WO-A-2006/061142
- JP-A- S62 178 505
- US-A1- 2004 052 749

## Description

### Field of the invention

This invention relates to a new cosmetic use of glycogen as a velvet agent in a cosmetic composition for skin application.

### State of the Art

Glycogen is a polysaccharide of animal origin mainly comprised of molecules of D-glucose linked together by α-1-4 glucoside bonds with branches every five-ten glucose units comprising glucoside α-1-6 bonds. The frequency and level of branching of the glycogen varies in relation to the animal species from which it has been obtained. The molecular weight of natural glycogen is of the order of 10⁶-10⁷ Dalton. In nature glycogen is always bound to a protein, glycogenin, an enzyme correlated with the process of cell glycogen synthesis.

Glycogen extracted from natural sources is used in the cosmetics industry as an emollient and hydrating agent, as described in JP-A-62-178 505 and JP-A-63-290 809, as an anti-ageing agent as described in US 5.093.109 and JP-A-2003-335651, and as a humectant and lubricant in ophthalmic solutions as described in WO99/47120.

The quality of commercial glycogen depends on the presence in greater or lesser quantities of protein residues (measured in terms of quantity of nitrogen, expressed as ppm) and reducing sugars.

Patent EP 654.048 describes a high-quality glycogen having a very low nitrogen content (< 60 ppm) and reducing sugars content (< 0.25% by weight).

### Definitions

For the purposes of this description and the following claims, by the term "velvet" or "velvet effect" is meant the achievement of a sensory effect of a soft and smooth skin.

### Summary of the invention

The Applicant has surprisingly found that the use of glycogen in a cosmetic formulation for skin application imparts a velvet effect to the skin.

In a second aspect this invention relates to the cosmetic use of glycogen, having a low nitrogen and reducing sugars content, as a velvet agent in a cosmetic composition for skin application.

The Applicant has found that application of the cosmetic composition according to this invention to the skin makes it possible to achieve a velvet effect on the treated skin.

In particular, the velvet effect is manifested through a sensation of softness and smoothness perceived by the treated individual about three minutes after application of the cosmetic composition according to this invention.

The Applicant has also observed that the cosmetic composition according to this invention is rapidly absorbed by the skin and provides a lifting effect of firm and smooth skin.

Moreover, the Applicant has also observed that the cosmetic composition according to this invention imparts greater brightness and lustre to treated skin.

Finally, the Applicant has observed that the cosmetic composition according to this invention can be easily manufactured in industrial applications and is stable over time.

### Brief description of the figures

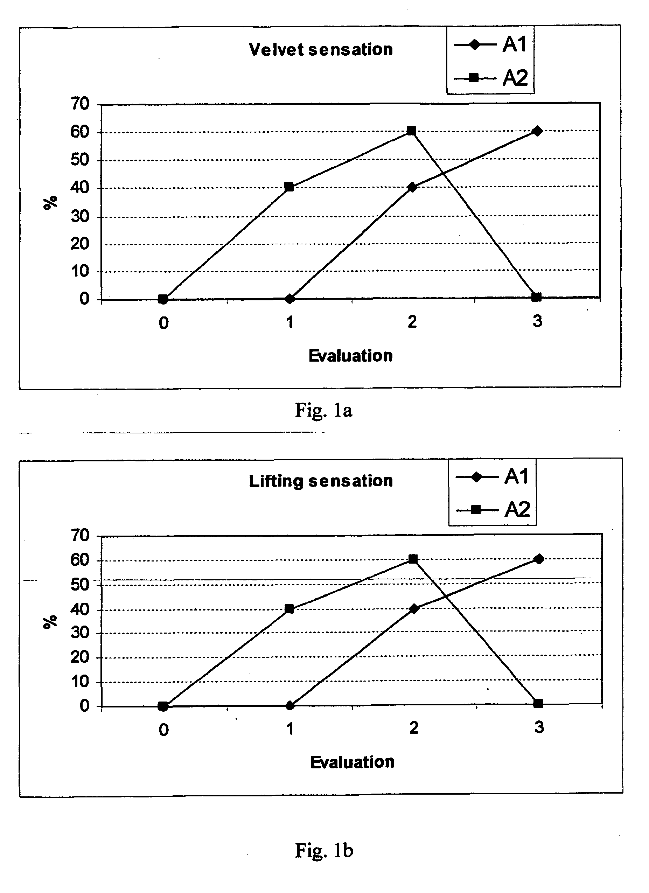

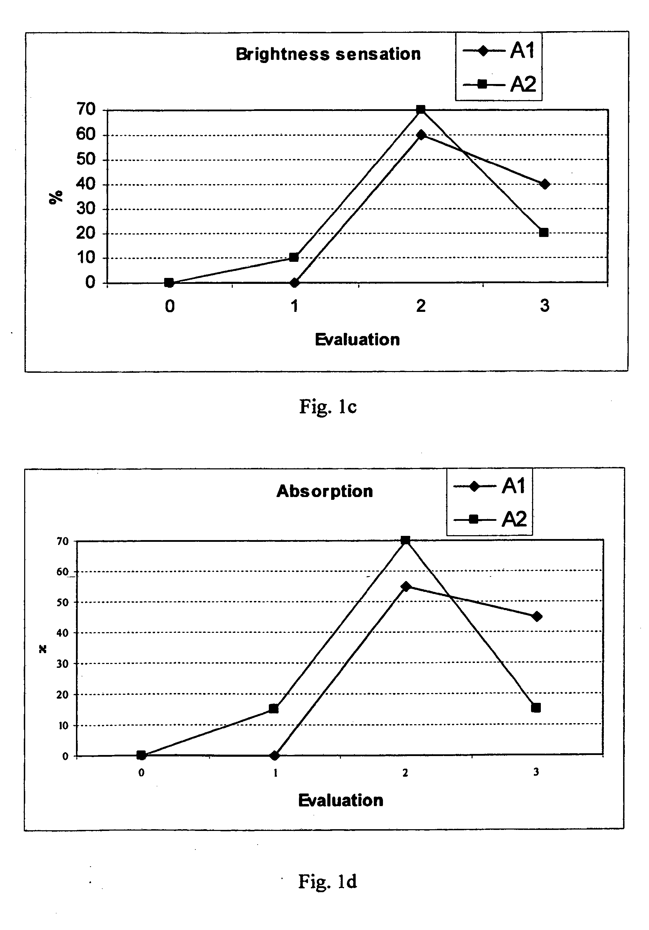

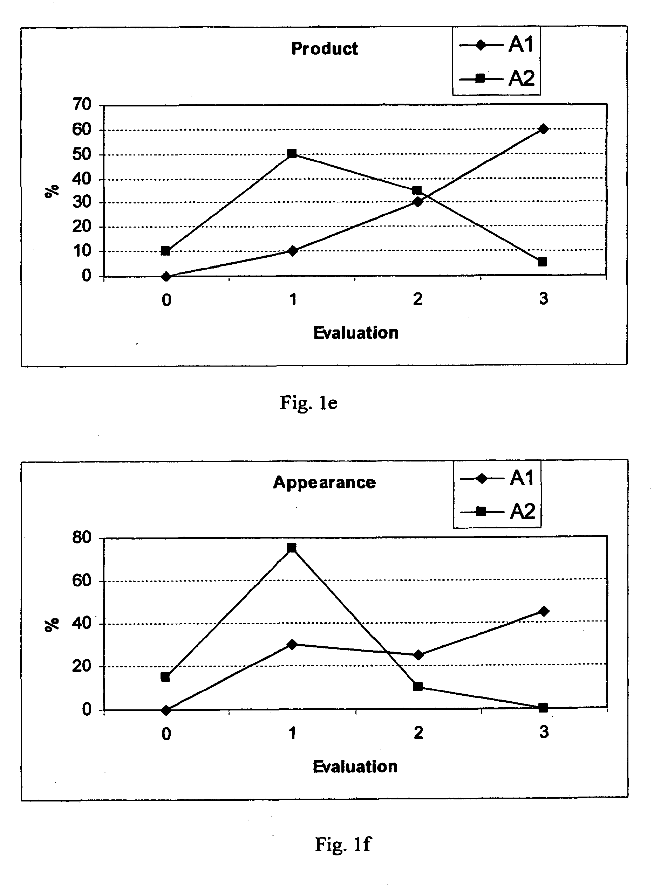

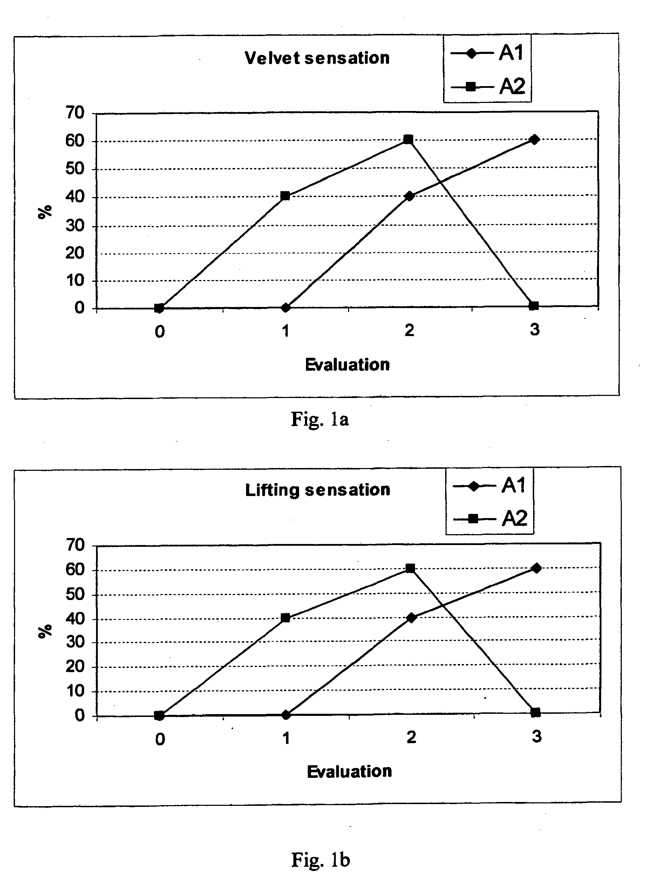

Figures 1a-1f show graphs comparing evaluations of the products in Example 1.

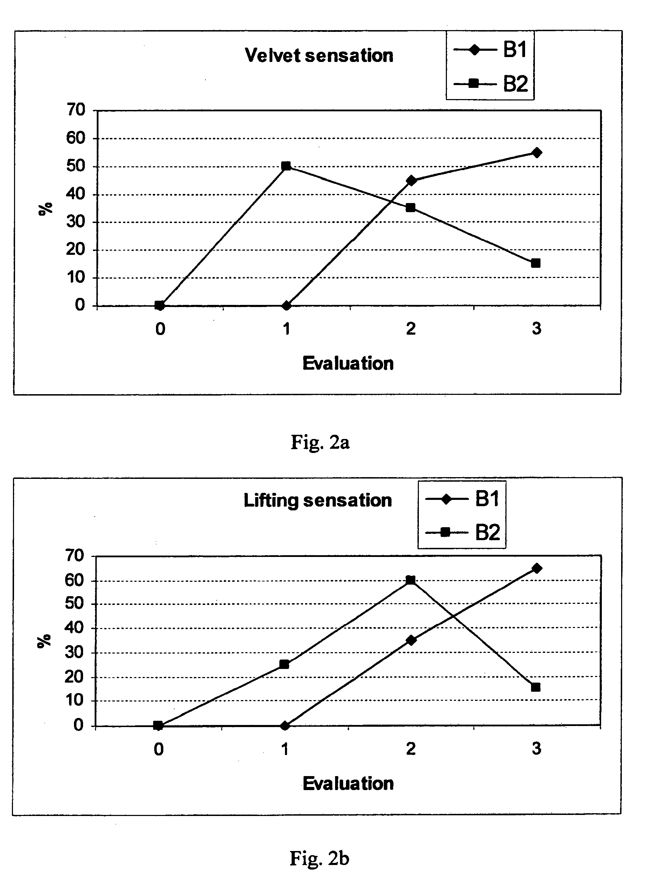

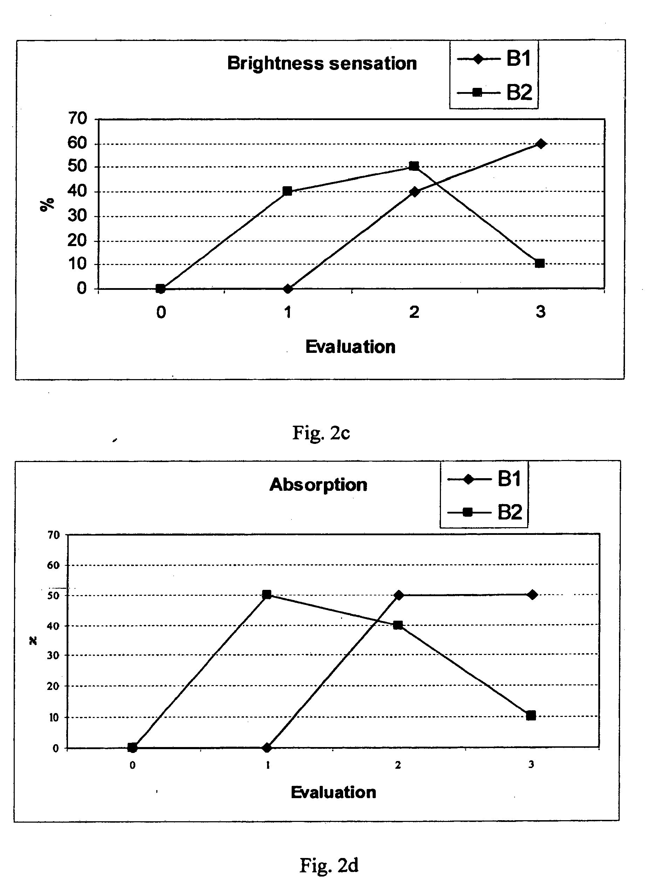

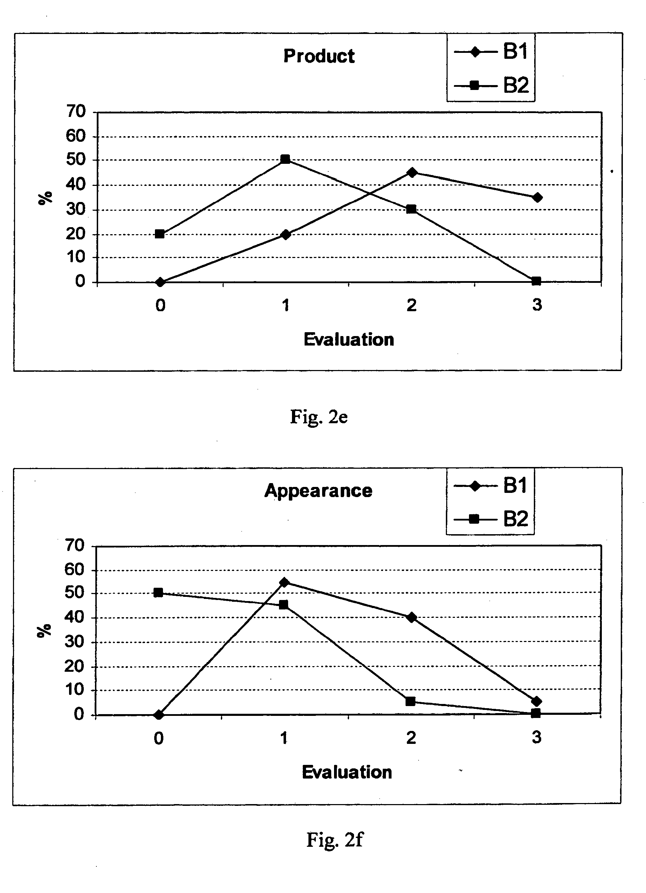

Figures 2a-2f show graphs comparing evaluations of the products in Example 2.

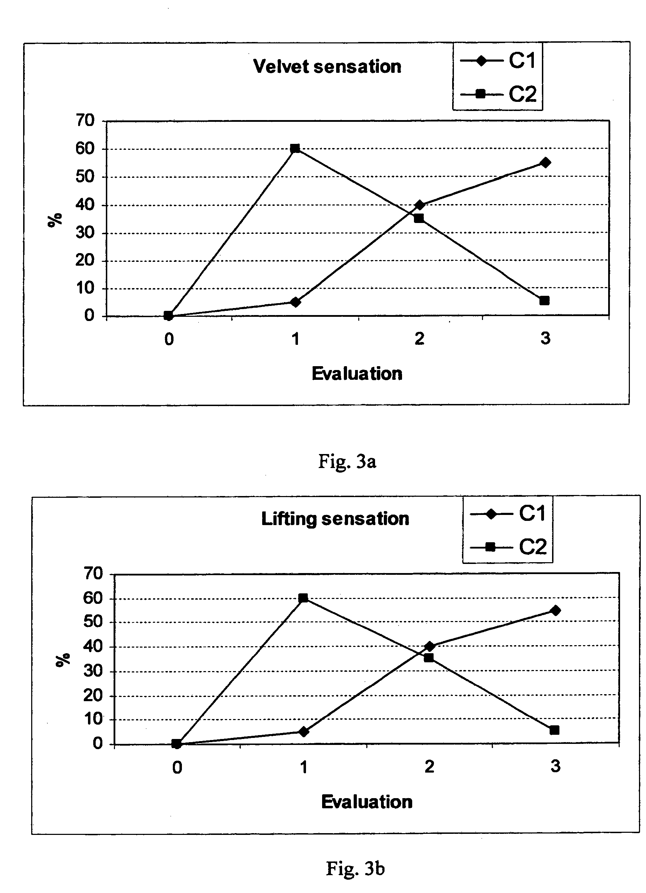

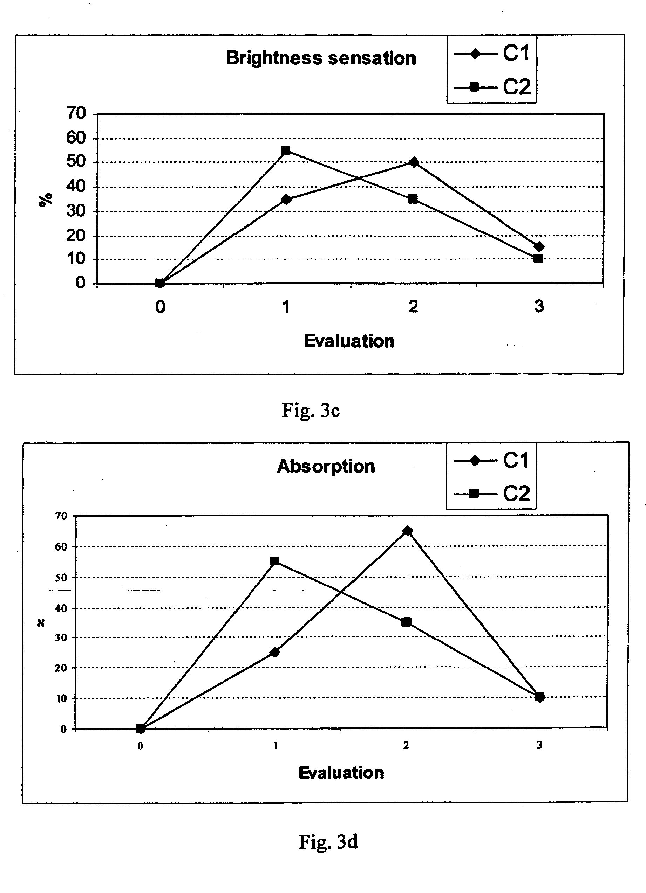

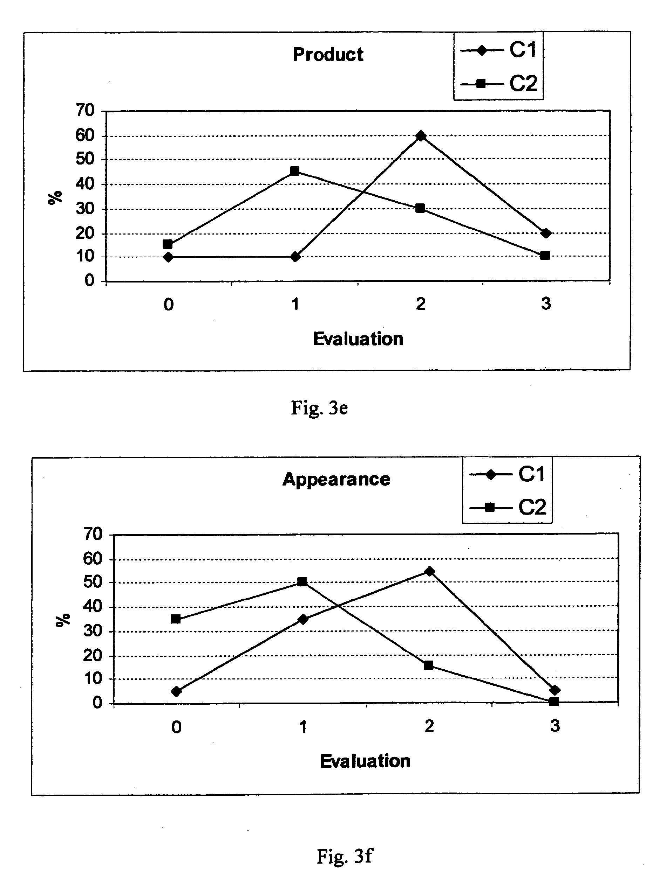

Figures 3a-3f show graphs comparing evaluations of the products in Example 3.

### Detailed description of the invention

The glycogen used in this invention is obtained from natural glycogen which may be extracted from animals ar fungi. Molluscs, in particular mussels (Mytilus edulis and Mytilus gallus provincialis) are a particularly useful source of glycogen because they are available in large quantities at low cost and contain a reasonable quantity of glycogen (on overage between 2.5% and 3.9% by weight). Other natural sources of glycogen include other bivalve molluscs such as clams, oysters, some species of gastropods or sea snails, such as slipper limpets (Crepidula fornicata), as well as organs of vertebrate animals which are rich in glycogen such as the liver and muscles.

The glycogen used in this invention may be used as such as obtained from extraction processes or may be treated in subsequent purification procedures. As already mentioned, the quality of a commercial glycogen depends on the presence of a greater or lesser quantity of protein residues (measured in terms of quantity of nitrogen expressed as ppm) and reducing sugars.

For the purposes of this invention a glycogen having a low content of reducing sugars and nitrogen is used. Examples of commercial products preferably used in this invention are glycogens produced and distributed by Sigma-Aldrich.

The glycogen used in this invention comprises less than 1 % by weight, and more preferably less than 0.25% by weight of reducing sugars, measured in accordance with the method by F.D. Snell and Snell, "Colorimetric Methods of Analysis", New York, 1954, vol. III, p. 204.

The glycogen used in this invention comprises less than 1000 and more preferably less than 100 ppm of nitrogen measured. using the Kjeldahl method.

Advantageously, the glycogen used in this invention is Polglumyt™ glycogen, the trade name of a deproteinated glycogen produced and distributed by A.C.R.A.F. S.p.A., Rome, Italy, and obtained in accordance with the purification procedure described in patent EP 654048 B1.

Preferably, the cosmetic composition according to this invention comprises a quantity of glycogen of between about 0.1 % and about 15%, more preferably between 0.5% and 10%, and even more preferably between 1 % and 5% by weight relative to the weight of the total formulation.

The cosmetic composition according to this invention comprises liquid or semi-solid formulations.

The liquid formulations for cosmetic use according to this invention comprise solutions, emulsions, microemulsions, lotions, foams, milks, oils, relaxants or suspensions of widely varying viscosity.

The liquid formulations may for example be aqueous solutions, hydroalcoholic solutions, oily solutions, emulsions obtained by dispersing an oily phase in an aqueous phase (oil-in-water) or vice-versa an aqueous phase in an oily phase (water-in-oil), and suspensions obtained by dispersing a dispersed phase comprising solid particles in a dispersing medium generally represented by an aqueous or oily liquid having a particular viscosity.

The semi-solid formulations for cosmetic use according to this invention comprise creams, gels, ointments, pastes, cream-gels, sticks and waxes.

The formulations for cosmetic use of this invention may comprise various cosmetically-acceptable additives or vehicles which are useful in the preparation of cosmetic products and known to those skilled in the art such as, for example, emulsifiers, hydrating agents, solvents, emollients, stabilisers, viscosity agents, preservatives, lubricants, sequestrating or chelating agents, fillers, fragrances, perfumes, absorbents, colouring agents and opacifiers, antioxidants, plant extracts and oils, vitamins, protective substances, essential oils, keratin-active substances and amino acids.

The liquid formulations for cosmetic use according to this invention preferably comprise at least one solvent, at least one hydrating agent, at least one sequestering agent, and at least one preservative.

The semi-solid formulations for cosmetic use according to this invention preferably comprise at least one solvent, at least one emulsifier, at least one viscosity agent, at least one hydrating agent, at least one emollient, and at least one preservative.

Suitable solvent additives comprise, for example, water, alcohols, ketones (such as acetone and methylisobutyl ketone), glycols (such as ethylene glycol, propylene glycol and butylene glycol), polyethylene glycols (such as PEG-40, PEG-50, PEG-60), alkyl acetates (such as amyl acetate, isopropyl acetate, butyl acetate), paraffins and isoparaffins, cycloalkyls (such as cyclohexane), glycerine, natural and synthetic oils, natural and synthetic triglycerides.

Depending upon the solvent used we can make a distinction between aqueous formulations and non-aqueous or water-free formulations.

In aqueous formulations the water represents the main component of the cosmetic composition and can even amount to a quantity of up to 99% by weight relative to the weight of the total formulation. Aqueous formulations contain a quantity of water of preferably between 25% and 95%, preferably between 50% and 90% by weight relative to the weight of the total formulation.

The aqueous formulations of the cosmetic composition according to this invention may preferably comprise a total quantity of non-aqueous solvents of between about 0.1 % and about 60%, more preferably between 1% and 40% and even more preferably between 5% and 35% by weight relative to the weight of the total formulation.

In non-aqueous or water-free formulations, water is absent and the total quantity of solvent additives other than water is between about 1% and about 99%, preferably between 25% and 95%, preferably between 50% and 90% by weight relative to the weight of the total formulation. The expression "water is absent" means that the quantity of water is less than 0.01 % by weight relative to the weight of the total formulation.

Suitable emulsifying additives may be non-ionic, cationic, anionic and amphoteric surfactants, or combinations thereof. Useful examples of emulsifiers are, for example, sorbitan, long-chain ethoxylated alcohols, alkyl polyglycosides, soaps, alkyl sulfates, such as for example sodium cetyl stearyl sulfate, monoalkyl and dialkyl phosphates, alkyl sulfonates, hydrogenated castor oil, acyl isothionates, saccharose esters, betain, lecithin, quaternary ammonium salts, alkyl oleates, glycerides such as for example caprylocaproyl polyoxylglycerides (caprylocaproyl macrogolglycerides) and emulsifying agents from olive oil.

The cosmetic composition according to this invention preferably comprises a total quantity of emulsifiers of between about 0.1 % and about 60%, more preferably between 0.5% and 25%, and even more preferably between 0.5% and 10% by weight relative to the weight of the total formulation.

Useful viscosity agents are represented for example by xanthan gum, hydroxypropylcellulose, hydroxyethylcellulose, carbopol, carrageenans, poloxamers and acacia gum.

The cosmetic composition according to this invention preferably comprises a total quantity of viscosity agents of between about 0.1% and about 25%, more preferably between 0.5% and 10%, and even more preferably between 0.5% and 5% by weight relative to the weight of the total formulation.

Additives having a hydrating action are for example urea, allantoin, hyaluronic acid and its derivates, glycerine, amino acids, acetyl monoethanolamide, butoxypropanol, butyl glycol, low molecular weight polyethylene glycols (such as PEG-40, PEG-50, PEG-60), aloe, mallow, and sorbitol.

The cosmetic composition according to this invention preferably comprises a total quantity of hydrating agents of between about 0.05% and about 25%, more preferably between 0.5% and 10%, and even more preferably between 0.1% and 5% by weight relative to the weight of the total formulation.

Suitable emollient additives include, for example, lanolin, almond oil, olive oil, hydrogenated castor oil, microcrystalline wax, polydimethylsiloxane (dimethicone), polymethylphenylsiloxane, polymers of glycol and silicone, mineral oils, paraffin, ozokerite, ceresine, triglyceride esters, monoglyceride acetylates, ethoxylated glycerides, alkyl esters of fatty acids, fatty acids, long-chain alcohols, sterols, beeswax, polyhydric alcohols, polyesters, and fatty acid amides.

The cosmetic composition according to this invention preferably comprises a total quantity of emollients of between about 0.1 % and about 25%, more preferably between 0.5% and 10%, and even more preferably between 0.5% and 5% by weight relative to the weight of the total formulation.

Suitable preservative additives include, for example, alcohols such as ethanol, phenoxyethanol and benzyl alcohol, methyl and propyl parahydroxybenzoate, hydroxyanisole butylate (BHA), sorbates, urea derivates and isothiazolinones.

The cosmetic composition according to this invention preferably comprises a total quantity of preservatives of between about 0.01 % and about 2.00%, more preferably between 0.05% and 1.00%, and even more preferably between 0.1% and 0.5% by weight relative to the weight of the total formulation.

Sequestrating or chelating additives include EDTA, HEDTA, alkyl oxalates, lithium or potassium oxalate, sodium or potassium pyrophosphate. The cosmetic composition according to this invention preferably comprises a total quantity of sequestrating or chelating agents of between about 0.01 % and about 20%, more preferably between 0.05% and 10%, and even more preferably between 0.1% and 5% by weight relative to the weight of the total formulation.

Suitable stabilising additives are, for example, long-chain alcohols (such as cetyl alcohol, stearyl alcohol) and mixtures thereof, high molecular weight polyethylene glycols (such as PEG-9000 and PEG-14000) and polyvinyl pyrrolidones (such as povidone).

The cosmetic composition according to this invention preferably comprises a total quantity of stabilising agents of between about 0.1 % and about 25%, more preferably between 0.5% and 15%, and even more preferably between 1% and 10% by weight relative to the weight of the total formulation.

Opacifiers are for example zinc or aluminium oxide, titanium or zinc dioxide , alumina, mica, aluminium salts of fatty acids, and chalk.

The colouring agents preferably used are readily washable watersoluble colouring agents which do not stain the skin or leave residues such as, for example, Acid Blue 3 C.I.42051 Acid Blue 9 C.I.42090, Acid Blue 74 C.I.73015, Pigment Blue 15 C.I.74160, Acid Yellow 3 C.I.47005, Food Grade Yellow 3 C.I.15985, Acid Yellow 23 C.I.19140, Acid Yellow 73 C.I.45350, Acid Red 14 C.I.14720, Acid Red 18 C.I.16255, Acid Red 27 C.I.16185, Acid Red 51 C.I.45430, Acid Green 1 C.I.10020, Acid Green 25 C.I.61570, and mixtures thereof.

The cosmetic composition according to this invention preferably comprises a total quantity of opacifiers and colouring agents of between about 0.01 % and about 15%, more preferably between 0.05% and 5% by weight relative to the weight of the total formulation.

The following examples serve to illustrate the invention without however limiting it.

### EXAMPLE 1

Two body cream formulations were prepared using the ingredients in Table 1 below. The quantities stated in Table 1 are percentages by weight (g/100g).

**TABLE 1**

| | A1 (invention) | A2 (comparison) |
|---|---|---|
| Polglumyt™ | 2 | - |
| Methyl p-hydroxybenzoate | 0.18 | 0.18 |
| Propyl p-hydroxybenzoate | 0.02 | 0.02 |
| Lanette O (cetyl stearyl alcohol) | 3 | 3 |
| Lanette E (sodium cetyl stearyl sulfate) | 0.5 | 0.5 |
| Dimethicone 300 cps | 0.5 | 0.5 |
| Allantoin | 0.2 | 0.2 |
| Purified water q.s. | 100 | 100 |

### EXAMPLE 2

Two face cream formulations were prepared using the ingredients in Table 2 below. The quantities stated in Table 2 are percentages by weight (g/100g).

**TABLE 2**

| | B1 (invention) | B2 (comparison) |
|---|---|---|
| Polglumyt™ | 2 | - |
| Methyl p-hydroxybenzoate | 0.18 | 0.18 |
| Propyl p-hydroxybenzoate | 0.02 | 0.02 |
| Lanette O (cetylstearyl alcohol) | 5 | 5 |
| Lanette E (sodium cetylstearyl sulfate) | 0.5 | 0.5 |
| Dimethicone 300 cps | 0.5 | 0.5 |
| Allantoin | 0.2 | 0.2 |
| Purified water q.s. | 100 | 100 |

### EXAMPLE 3

Two face tonic formulations were prepared using the ingredients in Table 3 below. The quantities stated in Table 3 are percentages by weight (g/100g).

**TABLE 3**

| | C1 (invention) | C2 (comparison) |
|---|---|---|
| Polglumyt™ | 2 | - |
| Butylene glycol | 20 | 20 |
| Glycerine | 10 | 10 |
| PEG 60 | 2 | 2 |
| Hydrogenated castor oil | 2 | 2 |
| Sodium hyaluronate | 0.1 | 0.1 |
| Disodium EDTA | 0.1 | 0.1 |
| Phenoxyethanol | 0.5 | 0.5 |
| Methyl p-hydroxybenzoate | 0.12 | 0.12 |
| Perfume | 0.2 | 0.2 |
| Purified water q.s. | 100 | 100 |

### EXAMPLE 4

The formulations in Examples 1 to 3 were subjected to evaluation according to the following procedure.

20 persons of the female sex aged between twenty and fifty years were selected. Instructions for the qualitative definition of perceived sensations specified in Table 4 below were provided to the persons included in the study.

**TABLE 4**

| Parameter | Description |
|---|---|
| Velvet sensation | Sensation of soft and smooth skin |
| Lifting sensation | Sensation of smooth and firm skin |
| Brightness sensation | Sensation of bright and lustrous skin |

The persons involved in the study were instructed to define the aforesaid perceptions quantitatively on the basis of the evaluation scale shown in Table 5 below.

**TABLE 5**

| Evaluation / Score | Description |
|---|---|
| None / (0) | Non-existent sensation |
| Low / (1) | Difficult to detect sensation |
| Medium / (2) | Easily detectable sensation |
| High / (3) | Very obvious sensation |

The persons involved in the study were also instructed to define the ease of absorption of the formulation under test in accordance with Table 6 below.

**TABLE 6**

| Evaluation / Score | Description |
|---|---|
| None / (0) | Time over 3 minutes |
| Low / (1) | Absorption within 3 minutes |
| Medium / (2) | Absorption within 2 minutes |
| High / (3) | Absorption within 1 minute |

Finally the persons included in the study were asked to provide an overall assessment of the product and the appearance of the product in accordance with Table 7 below.

**TABLE 7**

| Evaluation / Score | Description |
|---|---|
| 0 | Unacceptable |
| 1 | Acceptable |
| 2 | Good |
| 3 | Excellent |

In order to evaluate the sensory parameters of absorption and the overall assessments of the product and its appearance the assessments for the two formulations were compared using the signed-rank Wilcoxon statistical method.

Formulations A1, B1 and C1 containing Polglumyt™ glycogen formulations according to this invention were compared with the corresponding formulation A2, B2 and C2 without glycogen in accordance with a completely random cross double-blind experimental design. Three study sessions were carried out, one for each formulation. Each study session lasted one day. The formulations were applied by the individuals to the upper part of the forearm. The cream formulations were applied by hand spreading a quantity of approximately 3 grams. The tonic formulations were applied using a wad of cotton wool soaked in approximately 4 ml of solution. The area treated was massaged until the product was completely absorbed, for a time not exceeding three minutes.

The percent results and the value p of the statistical analysis of the evaluations made by the persons involved in the study for each parameter are summarised in the following tables. Table 8 relates to the results of the body cream products in Example 1. Table 9 relates to the results for the face cream products in Example 2. Table 10 relates to the results of the face tonic products in Example 3.

**TABLE 8**

| BODY CREAM | | | | | | |
|---|---|---|---|---|---|---|
| Parameter | Product | Evaluation % | | | | Value of p |
| | | 0 | 1 | 2 | 3 | |
| Velvet sensation | A1 | - | - | 40 | 60 | 0.074 |
| | A2 | - | 40 | 60 | - | |
| Lifting sensation | A1 | - | - | 40 | 60 | <0.0001 |
| | A2 | - | 40 | 60 | - | |
| Brightness sensation | A1 | - | - | 60 | 40 | NS |
| | A2 | - | 10 | 70 | 20 | |
| Absorption | A1 | - | - | 55 | 45 | 0.078 |
| | A2 | - | 15 | 70 | 15 | |
| Product | A1 | - | 10 | 30 | 60 | 0.0002 |
| | A2 | 10 | 50 | 35 | 5 | |
| Appearance | A1 | - | 30 | 25 | 45 | 0.0002 |
| | A2 | 15 | 75 | 10 | - | |

| | | | | | | |
|---|---|---|---|---|---|---|
| NS : Not significant | | | | | | |

All the values (with the exception of the brightness sensation) showed a statistically significant difference in favour of the formulation according to this invention.

**TABLE 9**

| FACE CREAM | | | | | | |
|---|---|---|---|---|---|---|
| Parameter | Product | Evaluation % | | | | Value of p |
| | | 0 | 1 | 2 | 3 | |
| Velvet sensation | B1 | - | - | 45 | 55 | 0.0031 |
| | B2 | - | 50 | 35 | 15 | |
| Lifting sensation | B1 | - | - | 35 | 65 | 0.0015 |
| | B2 | - | 25 | 60 | 15 | |
| Brightness sensation | B1 | - | - | 40 | 60 | 0.0002 |
| | B2 | - | 40 | 50 | 10 | |
| Absorption | B1 | - | - | 50 | 50 | 0.0011 |
| | B2 | - | 50 | 40 | 10 | |
| Product | B1 | - | 20 | 45 | 35 | 0.0013 |
| | B2 | 20 | 50 | 30 | - | |
| Appearance | B1 | - | 55 | 40 | 5 | 0.0007 |
| | B2 | 50 | 45 | 5 | - | |

All the values showed a statistically significant difference in favour of the formulation according to this invention.

**TABLE 10**

| FACE TONIC | | | | | | |
|---|---|---|---|---|---|---|
| Parameter | Product | Evaluation % | | | | Value of p |
| | | 0 | 1 | 2 | 3 | |
| Velvet sensation | C1 | - | 5 | 40 | 55 | 0.0022 |
| | C2 | - | 60 | 35 | 5 | |
| Lifting sensation | C1 | - | 5 | 40 | 55 | 0.273 |
| | C2 | - | 60 | 35 | 5 | |
| Brightness sensation | C1 | - | 35 | 50 | 15 | NS |
| | C1 | - | 55 | 35 | 10 | |
| Absorption | C1 | - | 25 | 65 | 10 | NS |
| | C2 | - | 55 | 35 | 10 | |
| Product | C1 | 10 | 10 | 60 | 20 | NS |
| | C2 | 15 | 45 | 30 | 10 | |
| Appearance | C1 | 5 | 35 | 55 | 5 | 0.0074 |
| | C2 | 35 | 50 | 15 | - | |

| | | | | | | |
|---|---|---|---|---|---|---|
| NS : Not significant | | | | | | |

The values for velvet and lifting sensation and the overall values relating to the appearance of the product showed a statistically significant difference in favour of the formulation according to this invention.

### EXAMPLE 5

A lipstick formulation was prepared using the ingredients in Table 11 below. The quantities stated in Table 11 are percentages by weight (g/100g).

**TABLE 11**

| Ingredient | Quantity |
|---|---|
| Polglumyt™ | 2 |
| Vaseline oil | 28 |
| Microcrystalline wax | 13 |
| Paraffin | 13.4 |
| Hydrogenated coconut oil | 13 |
| Beeswax | 6 |
| Hydrogenated lanolin | 10 |
| Propyl p-hydroxybenzoate | 0.15 |
| Hydroxyanisol butylate (BHA) | 0.05 |
| Titanium dioxide | 1.20 |
| Zinc dioxide | 1.20 |
| UVA filter | 2 |
| UVB filter | 8 |
| Vitamin E acetate | 2 |

### EXAMPLE 6

An aftershave formulation was prepared using the ingredients in Table 12 below. The quantities stated in Table 12 are percentages by weight (g/100g).

**TABLE 12**

| Ingredient | Quantity |
|---|---|
| Polglumyt™ | 2 |
| Water | 30 |
| Glycerol | 10 |
| Jojoba oil | 10 |
| Sorbitan oleate | 5 |
| Cetearyl olivate | 10 |
| Propylene glycol | 25 |
| Hydrogenated castor oil | 4 |
| Hydrogenated lecithin | 2.8 |
| Pantenol | 0.2 |
| Lactic acid | 0.1 |
| Tocopherol | 0.05 |
| Ascorbyl palmitate | 0.05 |
| Phenoxyethanol | 0.5 |
| Benzyl alcohol | 0.2 |
| Potassium sorbate | 0.1 |

### EXAMPLE 7

A body foam formulation (mousse type) was prepared using the ingredients in Table 13 below. The quantities stated in Table 13 are percentages by weight (g/100g).

**TABLE 13**

| Ingredient | Quantity |
|---|---|
| Polglumyt™ | 2 |
| Caprylocaproyl polyoxylglycerides (Caprylocaproyl macrogolglycerides) | 28 |
| Propylene glycol monolaurate | 10 |
| Propylene glycol dicaprylocaproate | 2.5 |
| Hydrogenated castor oil | 15 |
| Methyl p-hydroxybenzoate | 0.12 |
| Water q.s. | 100 |

### EXAMPLE 8

A hair lotion formulation was prepared using the ingredients in Table 14 below. The quantities stated in Table 14 are percentages by weight (g/100g).

**TABLE 14**

| Ingredient | Quantity |
|---|---|
| Polglumyt™ | 2 |
| Water | 58 |
| Glycerol | 10 |
| Olive oil esters | 6 |
| Sorbitan oleate | 5 |
| Cetearyl olivate | 3 |
| Betaine | 10 |
| Hydroxyethylcellulose | 4 |
| Pantenol | 1 |
| Lactic acid | 0.1 |
| Tocopherol | 0.05 |
| Ascorbyl palmitate | 0.05 |
| Phenoxyethanol | 0.5 |
| Benzyl alcohol | 0.2 |
| Potassium sorbate | 0.1 |

### EXAMPLE 9

A sun oil formulation was prepared using the ingredients in Table 15 below. The quantities stated in Table 15 are percentages by weight (g/100g).

**TABLE 15**

| Ingredient | Quantity |
|---|---|
| Polglumyt™ | 2 |
| Caprylic and capric acid esters (Cetiol™ LC) | 20 |
| Caprylic and capric acid triglyceride (Myritol™318) | 40 |
| 2-octyldodecanol | 36 |
| UV filter | 2 |

## Claims

1. Cosmetic use of a glycogen containing less than 1,000 ppm of nitrogen and less than 1% by weight of reducing sugars as a velvet agent (i.e. an agent to obtain the sensory effect of a soft and smooth skin) in a cosmetic composition for skin application.

2. Cosmetic use according to Claim 1, **characterised in that** the said glycogen comprises less than 100 ppm of nitrogen and less than 0.25% by weight of reducing sugars.

3. Cosmetic use according to any one of Claims 1 and 2, **characterised in that** the said composition comprises a quantity of the said glycogen of between about 0.1% and about 15% by weight relative to the weight of the total formulation in at least one cosmetically-acceptable vehicle.

4. Cosmetic use according to Claim 3, **characterised in that** the said composition comprises a quantity of the said glycogen of between 0.5% and 10% by weight relative to the weight of the total formulation.

5. Cosmetic use according to Claim 3, **characterised in that** the said composition comprises a quantity of the said glycogen of between 1 % and 5% by weight relative to the weight of the total formulation.

6. Cosmetic use according to claim 3, **characterised in that** the said cosmetically-acceptable vehicle is selected from the group comprising emulsifiers, hydrating agents, solvents, emollients, stabilisers, viscosity agents, preservatives, lubricants, sequestrating or chelating agents, fillers, fragrances, perfumes, absorbents, colouring agents and opacifiers, antioxidants, plant extracts and oils, vitamins, protective substances, essential oils, keratin-active substances and amino acids.

7. Cosmetic use according to any one of the preceding claims, **characterised in that** the said composition comprises liquid or semi-solid formulations.

8. Cosmetic use according to Claim 7, **characterised in that** the said liquid formulation comprises at least one solvent, at least one hydrating agent, at least one sequestering agent, and at least one preservative.

9. Cosmetic use according to Claim 7, **characterised in that** the said semi-solid formulation comprises at least one solvent, at least one emulsifier, at least one viscosity agent, at least one hydrating agent, at least one emollient, and at least one preservative.

10. Cosmetic use according to any one of Claims 6 to 9, **characterised in that** the said solvent is selected from the group comprising water, alcohols, ketones, glycols, polyethylene glycols, alkyl acetates, isoparaffins, cycloalkyls, glycerine, natural and synthetic oils, natural and synthetic triglycerides.

11. Cosmetic use according to any one of Claims 6 to 9, **characterised in that** the said emulsifier is selected from the group comprising sorbitans, ethoxylated long-chain alcohols, alkyl polyglycosides, soaps, alkyl sulfates, monoalkyl and dialkyl phosphates, alkyl suffocates, hydrogenated castor oil, acyl isothionates, saccharose esters, betaine, lecithin, quaternary ammonium salts, alkyl oleates, glycerides and olive oil emulsifiers.

12. Cosmetic use according to any one of Claims 6 to 9, **characterised in that** the said viscosity agent is selected from the group comprising xanthan gum, hydroxypropyl cellulose, hydroxyethyl cellulose, carbopol, carrageenans, poloxamers and acacia gums.

13. Cosmetic use according to any one of Claims 6 to 9, **characterised in that** the said hydrating agent is selected from the group comprising urea, allantoin, hyaluronic acid and its derivatives, glycerine, amino acids, acetyl monoethanolamide, butoxypropanol, butyl glycol, low molecular weight polyethylene glycols, aloe, mallow and sorbitol.

14. Cosmetic use according to any one of Claims 6 to 9, **characterised in that** the said emollient is selected from the group comprising ianolin, almond oil, olive oil, hydrogenated castor oil, microcrystalline wax, polydimethylsiloxane (dimethicone), polymethylphenylsiloxane, polymers of glycol and silicone, mineral oils, paraffin, ozokerite, ceresine, triglyceride esters, monoglyceride acetylates, ethoxylated glycerides, alkyl esters of fatty acids, fatty acids, long-chain alcohols, sterols, beeswax, polyhydric alcohols, polyesters, and fatty acid amides.

15. Cosmetic use according to any one of Claims 6 to 9, **characterised in that** the said preservative is selected from the group comprising alcohols such as ethanol, phenoxyethanol and benzyl alcohol, methyl and propyl parahydroxy benzoate, hydroxyanisole butylate (BHA), sorbates, urea derivates, and isothiazolinones.

16. Cosmetic use according to any one of Claims 6 to 9, **characterised in that** the said sequestering agent is selected from the group comprising EDTA, HEDTA, alkyl oxalates, lithium or potassium oxalate, sodium or potassium pyrophosphate.

17. Cosmetic use according to any one of Claims 6 to 16, **characterised in that** the said composition comprises a quantity of water up to 99% by weight relative to the weight of the total formulation.

18. Cosmetic use according to Claim 17, **characterised in that** the said composition comprises a quantity of water of between 25% and 95%, preferably between 50% and 90% by weight relative to the weight of the total formulation.

19. Cosmetic use according to either of Claims 17 and 18, **characterised in that** the said composition comprises a total quantity of non-aqueous solvents of between about 0.1% and about 60%, more preferably between 1% and 40% by weight relative to the weight of the total formulation.

20. Cosmetic use according to any one of Claims 6 to 16, **characterised in that** the said composition comprises a quantity of water of less than 0.01 % by weight relative to the weight of the total formulation and a total quantity of non-aqueous solvents of between about 1% and about 99%, preferably between 25% and 95% by weignt relative to the weight of the total formulation.

21. Cosmetic use according to any one of Claims 6 to 16, **characterised in that** the said composition comprises a quantity of the said emulsifier of between about 0.1 % and about 60%, preferably between 0.5% and 25% by weight relative to the weight of the total formulation.

22. Cosmetic use according to any one of Claims 6 to 16, **characterised in that** the said composition comprises a quantity of the said viscosity agent of between about 0.1% and about 25%, preferably between 0.5% and 10% by weight relative to the weight of the total formulation.

23. Cosmetic use according to any one of Claims 6 to 16, **characterised in that** the said composition comprises a quantity of the said hydrating agent of between about 0.05% and about 25%, preferably between 0.5% and 10% by weight relative to the weight of the total formulation.

24. Cosmetic use according to any one of Claims 6 to 16, **characterised in that** the said composition comprises a quantity of the said emollient agent of between about 0.1 % and about 25%, preferably between 0.5% and 10% by weight relative to the weight of the total formulation.

25. Cosmetic use according to any one of Claims 6 to 16, **characterised in that** the said composition comprises a quantity of the said preservative of between about 0.01% and about 2.00%, preferably between 0.05% and 1.00% by weight relative to the weight of the total formulation.

26. Cosmetic use according to any one of Claims 6 to 16, **characterised in that** the said composition comprises a quantity of the said sequestering agent of between about 0.01% and about 20%, preferably between 0.05% and 10% by weight relative to the weight of the total formulation.

## Patentansprüche

1. Kosmetische Verwendung eines Glykogens, das weniger als 1.000 ppm Stickstoff und weniger als 1 Gew.-% reduzierende Zucker enthält, als Samtweichmittel (d.h. als Mittel zur Erlangung des Sinneseindrucks einer weichen und geschmeidigen Haut) in einer kosmetischen Zusammensetzung zur Anwendung auf der Haut.

2. Kosmetische Verwendung nach Anspruch 1, **dadurch gekennzeichnet dass** das Glykogen weniger als 100 ppm Stickstoff und weniger als 0,25 Gew.-% reduzierende Zucker enthält.

3. Kosmetische Verwendung nach einem der Ansprüche 1 und 2, **dadurch gekennzeichnet, dass** die Zusammensetzung eine Menge des Glykogens von etwa 0,1 bis etwa 15 Gew.- %, bezogen auf das Gewicht der Gesamtformulierung, in mindestens einem kosmetisch akzeptablen Träger enthält.

4. Kosmetische Verwendung nach Anspruch 3, **dadurch gekennzeichnet, dass** die Zusammensetzung eine Menge des Glykogens von 0,5 bis 10 Gew.-%, bezogen auf das Gewicht der Gesamtformulierung, enthält.

5. Kosmetische Verwendung nach Anspruch 3, **dadurch gekennzeichnet, dass** die Zusammensetzung eine Menge des Glykogens von 1 bis 5 Gew.-%, bezogen auf das Gewicht der Gesamtformulierung, enthält.

6. Kosmetische Verwendung nach Anspruch 3, **dadurch gekennzeichnet, dass** der kosmetisch akzeptable Träger ausgewählt ist unter Emulgatoren, Feuchtigkeitsmitteln, Lösungsmitteln, erweichenden Mitteln, Stabilisatoren, Viskositätsvermittlern, Konservierungsmitteln, Gleitmitteln, Sequestriermitteln oder Komplexierungsmitteln, Füllstoffen, Duftstoffen, Parfümstoffen, Absorbtionsmitteln, Farbstoffen, Opazifizierungsmitteln, Antioxidationsmitteln, Pflanzenextrakten und Ölen, Vitaminen, Schutzmitteln, ätherischen Ölen, Kreatin-aktiven Substanzen und Aminosäuren.

7. Kosmetische Verwendung nach einem der vorhergehenden Ansprüche, **dadurch gekennzeichnet, dass** die Zusammensetzung flüssige oder halbfeste Formulierungen umfasst.

8. Kosmetische Verwendung nach Anspruch 7, **dadurch gekennzeichnet, dass** die flüssige Formulierung mindestens ein Lösungsmittel, mindestens ein Feuchtigkeitsmittel, mindestens ein Sequestriermittel und mindestens ein Konservierungsmittel enthält.

9. Kosmetische Verwendung nach Anspruch 7, **dadurch gekennzeichnet, dass** die halbfeste Formulierung mindestens ein Lösungsmittel, mindestens einen Emulgator, mindestens einen Viskositätsvermittler, mindestens ein Feuchtigkeitsmittel, mindestens ein erweichendes Mittel und mindestens ein Konservierungsmittel enthält.

10. Kosmetische Verwendung nach einem der Ansprüche 6 bis 9, **dadurch gekennzeichnet, dass** das Lösungsmittel unter Wasser, Alkoholen, Ketonen, Glykolen, Polyethylenglykolen, Alkylacetaten, Isoparaffinen, Cycloalkylen, Glycerin, natürlichen und synthetischen Ölen, natürlichen und synthetischen Triglyceriden ausgewählt ist.

11. Kosmetische Verwendung nach einem der Ansprüche 6 bis 9, **dadurch gekennzeichnet, dass** der Emulgator unter Sorbitanen, ethoxylierten langkettigen Alkoholen, Alkylpolyglycosiden, Seifen, Alkylsulfaten, Mono-und Dialkylphosphaten, Alkylsulfonaten, hydriertem Rizinusöl, Acylisothionaten, Saccharoseestem, Betain, Lecithin, quartären Ammoniumsalzen, Alkyloleaten, Glyceriden und Olivenölemulgatoren ausgewählt ist.

12. Kosmetische Verwendung nach einem der Ansprüche 6 bis 9, **dadurch gekennzeichnet, dass** der Viskositätsvermittler unter Xanthangummi, Hydroxypropylcellulose, Hydroxyethylcellulose, Carbopol, Karragheenanen, Poloxameren und Akaziengummi ausgewählt ist.

13. Kosmetische Verwendung nach einem der Ansprüche 6 bis 9, **dadurch gekennzeichnet, dass** das Feuchtigkeitsmittel unter Harnstoff, Allantoin, Hyaluronsäure und deren Derivaten, Glycerin, Aminosäuren, Acetylmonoethanolamid, Butoxypropanol, Butylglykol, niedermolekularen Polyethylenglykolen, Aloe, Malve und Sorbitol ausgewählt ist.

14. Kosmetische Verwendung nach einem der Ansprüche 6 bis 9, **dadurch gekennzeichnet, dass** das erweichende Mittel unter Lanolin, Mandelöl, Olivenöl, hydriertem Rizinusöl, mikrokristallinem Wachs, Polydimethylsiloxan (Dimethicon), Polymethylphenylsiloxan, Polymeren aus Glykol und Silikon, Mineralölen, Paraffin, Ozokerit, Ceresin, Triglyceridestern, Monoglyceridacetylaten, ethoxylierten Glyceriden, Alkylestem von Fettsäuren, Fettsäuren, langkettigen Alkoholen, Sterolen, Bienenwachs, mehrwertigen Alkoholen, Polyestern und Fettsäureamiden ausgewählt ist.

15. Kosmetische Verwendung nach einem der Ansprüche 6 bis 9, **dadurch gekennzeichnet, dass** das Konservierungsmittel unter Alkoholen wie Ethanol, Phenoxyethanol und Benzylalkohol, Methyl- und Propylparahydroxybenzoat, Butylhydroxyanisol (BHA), Sorbaten, Harnstoffderivaten und Isothiazolinonen ausgewählt ist.

16. Kosmetische Verwendung nach einem der Ansprüche 6 bis 9, **dadurch gekennzeichnet, dass** das Sequestriermittel unter EDTA, HEDTA, Alkyloxalaten, Lithium- oder Kaliumoxalat, Natrium- oder Kaliumpyrophosphat ausgewählt ist.

17. Kosmetische Verwendung nach einem der Ansprüche 6 bis 16, **dadurch gekennzeichnet, dass** die Zusammensetzung eine Menge Wasser von bis zu 99 Gew.-%, bezogen auf das Gewicht der Gesamtformulierung, enthält.

18. Kosmetische Verwendung nach Anspruch 17, **dadurch gekennzeichnet, dass** die Zusammensetzung eine Menge Wasser von 25% bis 95 Gew.-%, vorzugsweise von 50 bis 90 Gew.-%, bezogen auf das Gewicht der Gesamtformulierung, enthält.

19. Kosmetische Verwendung nach den beiden Ansprüchen 17 und 18, **dadurch gekennzeichnet, dass** die Zusammensetzung eine Gesamtmenge an nicht-wässrigen Lösungsmitteln von etwa 0,1 bis etwa 60 Gew.-%, vorzugsweise von 1 bis 40 Gew.-%, bezogen auf das Gewicht der Gesamtformulierung, enthält.

20. Kosmetische Verwendung nach einem der Ansprüche 6 bis 16, **dadurch gekennzeichnet, dass** die Zusammensetzung eine Menge von weniger als 0,01 Gew.-% Wasser, bezogen auf das Gewicht der Gesamtformulierung, und eine Gesamtmenge an nicht-wässrigen Lösungsmitteln von etwa 1 bis etwa 99 Gew.-%, vorzugsweise von 25 bis 95 Gew.-%, bezogen auf das Gewicht der Gesamtformulierung, enthält.

21. Kosmetische Verwendung nach einem der Ansprüche 6 bis 16, **dadurch gekennzeichnet, dass** die Zusammensetzung eine Menge des Emulgators von etwa 0,1 bis etwa 60 Gew.-%, vorzugsweise von 0,5 bis 25 Gew.-%, bezogen auf das Gewicht der Gesamtformulierung, enthält.

22. Kosmetische Verwendung nach einem der Ansprüche 6 bis 16, **dadurch gekennzeichnet, dass** die Zusammensetzung eine Menge des Viskositätsvermittlers von etwa 0,1 bis etwa 25 Gew.-%, vorzugsweise von 0,5% bis 10 Gew.-%, bezogen auf das Gewicht der Gesamtformulierung, enthält.

23. Kosmetische Verwendung nach einem der Ansprüche 6 bis 16, **dadurch gekennzeichnet, dass** die Zusammensetzung eine Menge des Feuchtigkeitsmittels von etwa 0,05 bis etwa 25 Gew.-%, vorzugsweise von 0,5 bis 10 Gew.-%, bezogen auf das Gewicht der Gesamtformulierung, enthält.

24. Kosmetische Verwendung nach einem der Ansprüche 6 bis 16, **dadurch gekennzeichnet, dass** die Zusammensetzung eine Menge des erweichenden Mittels von etwa 0,1 bis etwa 25 Gew.-%, vorzugsweise von 0,5% bis 10 Gew.-%, bezogen auf das Gewicht der Gesamtformulierung, enthält.

25. Kosmetische Verwendung nach einem der Ansprüche 6 bis 16, **dadurch gekennzeichnet, dass** die Zusammensetzung eine Menge des Konservierungsmittels von etwa 0,01 bis etwa 2,00 Gew.-%, vorzugsweise von 0,05 bis 1,00 Gew.-%, bezogen auf das Gewicht der Gesamtformulierung, enthält.

26. Kosmetische Anwendung nach einem der Ansprüche 6 bis 16, **dadurch gekennzeichnet, dass** die Zusammensetzung eine Menge des Sequestriermittels von etwa 0,01 bis etwa 20 Gew.-%, vorzugsweise von 0,05 bis 10 Gew.-%, bezogen auf das Gewicht der Gesamtformulierung, enthält.

## Revendications

1. Utilisation cosmétique d'un glycogène contenant moins de 1000 ppm d'azote et moins de 1 % en poids de sucres réducteurs, en tant qu'agent veloutant, c'est-à-dire un agent pour obtenir l'effet sensoriel d'une peau souple et lisse, dans une composition cosmétique pour application sur la peau.

2. Utilisation cosmétique selon la revendication 1, **caractérisée en ce que** ledit glycogène comprend moins de 100 ppm d'azote et moins de 0,25 % en poids de sucres réducteurs.

3. Utilisation cosmétique selon l'une quelconque des revendications 1 et 2, **caractérisée en ce que** ladite composition comprend une quantité dudit glycogène comprise entre environ 0,1 % et environ 15 % en poids, par rapport au poids de la formulation totale, dans au moins un véhicule acceptable du point de vue cosmétique.

4. Utilisation cosmétique selon la revendication 3, **caractérisée en ce que** ladite composition comprend une quantité dudit glycogène comprise entre 0,5 % et 10 % en poids, par rapport au poids de la formulation totale.

5. Utilisation cosmétique selon la revendication 3, **caractérisée en ce que** ladite composition comprend une quantité dudit glycogène comprise entre 1 % et 5 % en poids, par rapport au poids de la formulation totale.

6. Utilisation cosmétique selon la revendication 3, **caractérisée en ce que** ledit véhicule acceptable du point de vue cosmétique est choisi dans le groupe comprenant les émulsionnants, les agents hydratants, les solvants, les émollients, les stabilisants, les agents de viscosité, les conservateurs, les lubrifiants, les agents séquestrants ou chélatants, les charges, les fragrances, les parfums, les absorbants, les agents colorants et les opacifiants, les antioxydants, les extraits et huiles de plantes, les vitamines, les substances protectrices, les huiles essentielles, les substances actives sur la kératine, et les acides aminés.

7. Utilisation cosmétique selon l'une quelconque des revendications précédentes, **caractérisée en ce que** ladite composition comprend des formulations liquides ou semi-solides.

8. Utilisation cosmétique selon la revendication 7, **caractérisée en ce que** ladite formulation liquide comprend au moins un solvant, au moins un agent hydratant, au moins un agent séquestrant, et au moins un conservateur.

9. Utilisation cosmétique selon la revendication 7, **caractérisée en ce que** ladite formulation semi-solide comprend au moins un solvant, au moins un émulsionnant, au moins un agent de viscosité, au moins un agent hydratant, au moins un émollient, et au moins un conservateur.

10. Utilisation cosmétique selon l'une quelconque des revendications 6 à 9, **caractérisée en ce que** ledit solvant est choisi dans le groupe comprenant l'eau, les alcools, les cétones, les glycols, les polyéthylèneglycols, les acétates d'alkyle, les isoparaffines, les cycloalkyles, la glycérine, les huiles naturelles et synthétiques, les triglycérides naturels et synthétiques.

11. Utilisation cosmétique selon l'une quelconque des revendications 6 à 9, **caractérisée en ce que** ledit émulsionnant est choisi dans le groupe comprenant les sorbitans, les alcools éthoxylés à longue chaîne, les alkylpolyglycosides, les savons, les alkylsulfates, les phosphates monoalkyliques et dialkyliques, les alkylsulfonates, l'huile de ricin hydrogénée, les acylisothionates, les esters de saccharose, la bétaïne, la lécithine, les sels d'ammonium quaternaire, les oléates d'alkyle, les glycérides, et les émulsionnants à base d'huile d'olive.

12. Utilisation cosmétique selon l'une quelconque des revendications 6 à 9, **caractérisée en ce que** ledit agent de viscosité est choisi dans le groupe comprenant la gomme xanthane, l'hydroxypropylcellulose, l'hydroxyéthylcellulose, le carbopol, les carraghénanes, les poloxamères et la gomme arabique.

13. Utilisation cosmétique selon l'une quelconque des revendications 6 à 9, **caractérisée en ce que** ledit agent hydratant est choisi dans le groupe comprenant l'urée, l'allantoïne, l'acide hyaluronique et ses dérivés, la glycérine, les acides aminés, l'acétylmonoéthanolamide, le butoxypropanol, le butylglycol, les polyéthylèneglycols de faible masse moléculaire, l'aloès, la mauve et le sorbitol.

14. Utilisation cosmétique selon l'une quelconque des revendications 6 à 9, **caractérisée en ce que** ledit émollient est choisi dans le groupe comprenant la lanoline, l'huile d'amande, l'huile d'olive, l'huile de ricin hydrogénée, la cire microcristalline, le polydiméthylsiloxane (diméthicone), le polyméthylphényl-siloxane, les polymères de glycol et de silicone, les huiles minérales, la paraffine, l'ozocérite, la cérésine, les esters triglycéridiques, les monoglycérides acétylés, les glycérides éthoxylés, les esters alkyliques d'acides gras, les acides gras, les alcools à longue chaîne, les stérols, la cire d'abeille, les alcools polyvalents, les polyesters, et les amides d'acides gras.

15. Utilisation cosmétique selon l'une quelconque des revendications 6 à 9, **caractérisée en ce que** ledit conservateur est choisi dans le groupe comprenant les alcools tels que l'éthanol, le phénoxyéthanol et l'alcool benzylique, le parahydroxybenzoate de méthyle ou de propyle, l'hydroxyanisole butylée (BHA), les sorbates, les dérivés d'urée, et les isothiazolinones.

16. Utilisation cosmétique selon l'une quelconque des revendications 6 à 9, **caractérisée en ce que** ledit agent séquestrant est choisi dans le groupe comprenant l'EDTA, le HEDTA, les oxalates d'alkyle, l'oxalate de lithium ou de potassium, le pyrophosphate de sodium ou de potassium.

17. Utilisation cosmétique selon l'une quelconque des revendications 6 à 16, **caractérisée en ce que** ladite composition comprend une quantité d'eau allant jusqu'à 99 % en poids par rapport au poids de la formulation totale.

18. Utilisation cosmétique selon la revendication 17, **caractérisée en ce que** ladite composition comprend une quantité d'eau comprise entre 25 % et 95 %, de préférence entre 50 % et 90 % en poids par rapport au poids de la formulation totale.

19. Utilisation cosmétique selon l'une ou l'autre des revendications 17 et 18, **caractérisée en ce que** ladite composition comprend une quantité totale de solvants non aqueux comprise entre environ 0,1 % et environ 60 %, mieux encore entre 1 % et 40 % en poids par rapport au poids de la formulation totale.

20. Utilisation cosmétique selon l'une quelconque des revendications 6 à 16, **caractérisée en ce que** ladite composition comprend une quantité d'eau inférieure à 0,01 % en poids par rapport au poids de la formulation totale et une quantité totale de solvants non aqueux comprise entre environ 1 % et environ 99 %, de préférence entre 25 % et 95 % en poids par rapport au poids de la formulation totale.

21. Utilisation cosmétique selon l'une quelconque des revendications 6 à 16, **caractérisée en ce que** ladite composition comprend une quantité dudit émulsionnant comprise entre environ 0,1 % et environ 60 %, de préférence entre 0,5 % et 25 % en poids par rapport au poids de la formulation totale.

22. Utilisation cosmétique selon l'une quelconque des revendications 6 à 16, **caractérisée en ce que** ladite composition comprend une quantité dudit agent de viscosité comprise entre environ 0,1 % et environ 25 %, de préférence entre 0,5 % et 10 % en poids par rapport au poids de la formulation totale.

23. Utilisation cosmétique selon l'une quelconque des revendications 6 à 16, **caractérisée en ce que** ladite composition comprend une quantité dudit agent hydratant comprise entre environ 0,05 % et environ 25 %, de préférence entre 0,5 % et 10 % en poids par rapport au poids de la formulation totale.

24. Utilisation cosmétique selon l'une quelconque des revendications 6 à 16, **caractérisée en ce que** ladite composition comprend une quantité dudit agent émollient comprise entre environ 0,1 % et environ 25 %, de préférence entre 0,5 % et 10 % en poids par rapport au poids de la formulation totale.

25. Utilisation cosmétique selon l'une quelconque des revendications 6 à 16, **caractérisée en ce que** ladite composition comprend une quantité dudit conservateur comprise entre environ 0,01 % et environ 2,00 %, de préférence entre 0,05 % et 1,00 % en poids par rapport au poids de la formulation totale.

26. Utilisation cosmétique selon l'une quelconque des revendications 6 à 16, **caractérisée en ce que** ladite composition comprend une quantité dudit agent séquestrant comprise entre environ 0,01 % et environ 20 %, de préférence entre 0,05 % et 10 % en poids par rapport au poids de la formulation totale.
